# EUROPÄISCHE PATENTSCHRIFT

(11) **EP 1 871 445 B1**
(45) Veröffentlichungstag und Bekanntmachung des Hinweises auf die Patenterteilung: **24.02.2016**
(21) Anmeldenummer: 06742237.8
(22) Anmeldetag: 12.04.2006
(51) Int. Cl.: A61M 5/148

(54) **VORRICHTUNG ZUM ENTLEEREN DES INHALTS EINES TRANSFUSIONS-/INFUSIONSBEUTELS**
DEVICE FOR EMPTYING THE CONTENTS OF A TRANSFUSION/INFUSION SAC
DISPOSITIF DE VIDAGE D'UNE POCHE DE TRANSFUSION/PERFUSION

(30) Priorität: 15.04.2005 DE 202005006146 U
(43) Veröffentlichungstag der Anmeldung: 02.01.2008
(73) Patentinhaber: Transmed Medizintechnik GmbH & Co. KG, 33181 Bad Wünnenberg (DE)
(72) Erfinder: HENZE, Wolfgang, 59602 Rüthen (DE)
(74) Vertreter: Grosse, Wolf-Dietrich Rüdiger
(86) Internationale Anmeldenummer: PCT/DE2006/000652
(87) Internationale Veröffentlichungsnummer: WO 2006/108404

(56) Entgegenhaltungen:
- WO-A-99/22786
- GB-A- 1 206 605
- US-A- 4 090 514
- US-A- 4 552 153
- US-A- 4 735 613
- US-A- 5 053 011
- US-A- 5 346 477
- US-A- 5 776 105
- US-A- 5 891 097

## Beschreibung

Die Erfindung betrifft eine Vorrichtung zum Entleeren des Inhalts eines Transfusions-/Infusionsbeutels, umfassend einen Pumpball, ein Manometer, ein Schnellentlüftungsventil, ein Rückschlagventil und eine Druckblase, in die der Transfusions-/Infusionsbeutel eingewickelt wird, wobei diese Bauteile oder Schläuche miteinander verbindbar sind. Die erfindung ist in Anspruch 1 definiert.

Solche Transfusions-/Infusionsbeutel-Entleerungsvorrichtungen sind durch die US 5 776 105 A oder US 4 090 514 A, die einen Stand der Technik gemäß dem Oberbegriff des Anspruchs 1 beschreibt, bekannt geworden. Diese Vorrichtungen sind allerdings wenig bedienungsfreundlich und baulich aufwendig, weil die erforderlichen Funktionseinheiten räumlich entfernt voneinander angeordnet sind.

Aus der GB 1 206 605 A ist es bekannt, bei einer aufblasbaren Bandage zur Verwendung als Druckverband, als Staubinde zum Stillen des Blutflusses oder als Manschette zum Messen von Blutdrücken einen Druckregler vorzusehen, der in einem rohrartigen Grundkörper im Übergang zu einem Pump- bzw. Gummiball ein Auslassrückschlagventil aufweist. In die Wand des rohrartigen Grundkörpers sind weiterhin ein Überdruckventil und ein Ablassventil eingeschraubt. An dem von dem Pumpball entfernten oberen Ende ist der rohrartige Grundkörper zur Aufnahme eines aufsteckbaren Druckmessers bzw. Manometers ausgebildet.

Die bekannten Vorrichtungen bzw. Systeme, die sich von Einheiten zur Blutdruckmessung ablelten, besitzen nur geringe Strömungsquerschnitte und Entlüftungsdurchmesser (etwa 1 mm Durchmesser), was wegen der in den Schlauchverbindungen auftretenden Reibung der Luft das Aufpumpen erschwert, weil ein großer Strömungswiderstand vorliegt. Außerdem nimmt das Entlüften eine insbesondere im Notfall unerwünscht hohe Zeit in Anspruch und beträgt mit manueller Unterstützung beim Ausdrücken mindestens 20 bis 30 Sekunden. Zudem wird für die Druckblase neben dem Schlauchanschluß für den Pumpball ein zweiter, das Manometer aufweisender Schlauch erforderlich, um das Manometer vor Druckspitzen zu schützen, die ansonsten beim Aufpumpen mit jedem Pumpstoß auftreten würden. Auch diese Druckspitzen gehen auf die in den verwendeten Standardschläuchen hohen Reibungsverluste zurück.

Der Erfindung liegt daher die Aufgabe zugrunde, eine gattungsgemäße Vorrichtung ohne die genannten Nachteile zu schaffen, insbesondere ein einfacheres, weniger anstrengendes Aufpumpen und sehr viel schnelleres Entlüften zu ermöglichen sowie dabei gleichzeitig die Bedienungsfreundlichkeit zu verbessern und den Aufwand für die Baueinheit zu verringern.

Diese Aufgabe wird erfindungsgemäß durch eine Ventile-Manometer-Einheit gelöst, die in einem gemeinsamen Gehäuse integriert das Manometer und das Schnellentlüftungsventil aufweist, die über eine Rohrleitung miteinander verbunden sind, deren Innendurchmesser einen durchgängig einheitlich großen Strömungsquerschnitt wie auf beidendige Anschlussabschnitte der Rohrleitung aufzusteckende Schlauchenden einerseits des Pumpballs bzw. andererseits der Druckblase besitzt, wobei in der Rohrleitung zwischen dem Pumpball-Anschlussabschnitt und dem Manometer ein Drehzylinder als Mehrwege-Schnellentlüftungsventil angeordnet ist, das in den Ventilstellungen zum Aufpumpen oder zum Entlüften mit dem durchgängig einheitlich großen Strömungsquerschnitt oder Innendurchmesser wie die Rohrleitung, die das Manometer und das Schnellentlüftungsventil miteinander verbindet, ausgebildet ist, und in dem Pumpball-Anschlussabshnitt das Rückschlagventil vorgesehen ist.

Abgesehen davon, daß eine als kompaktes Kunststoff-Spritzgußteil realisierbare Ventil-Manometer-Einheit mit allen funktionswesentlichen Komponenten vorliegt, an die dann lediglich noch der Pumpball bzw. Handpumpball und der Gummischlauch der Druckblase angeschlossen zu werden braucht, lassen sich weitere Vorteile gleichzeitig erreichen. Die durchgängig einheitlich groß geschaffenen Strömungsquerschnitte aller luftführenden Strecken, die einen Innendurchmesser der Rohrleitung und der Anschlüsse des Pumpballs und der Druckblase von vorzugsweise mindestens 8 mm besitzen, den auch das Schnellentlüftungsventil voll frelgibt, erlauben ein leistungsfähiges, zügiges Austauschen der Konserven bzw. Transfusions-/Infusionsbeutel, wenn im Notfall davon mehrere schnellstmöglich aufeinanderfolgend benötigt werden. Die Entlüftungszeit liegt bei lediglich 1 bis 2 Sekunden und ist damit etwa 10 mal schneller als bei den bisher üblichen Druckblasen/-manschetten.

Zum Entlüften braucht lediglich der Drehzylinder der Ventile-Manometer-.Einheit mittels eines Drehhebels in die für den Bediener durch eine äußere Kennzeichnung ersichtliche Auf-Stellung verdreht zu werden, womit die Druckluft aus der Druckblase vorbei am Manometer durch eine seitliche Öffnung im Schnellentlüftungsventil nach außen abströmen kann.

Aufgrund des erfindungsgemäß durchgängig großen, d.h. über den Durchmessern von Standardschläuchen liegenden Strömungsquerschnitts treten kaum noch Reibungsverluste und Druckspitzen auf, so daß für die Druckblase/-manschette kein separater, zweiter Schlauch für das Manometer mehr erforderlich ist. Die erfindungsgemäße Ventile-Manometer-Einheit ist gleichwohl durch Reduzierstücke abwärtskompatibel zu Systemen mit Standardschlauchdurchmessern. Allerdings sind dann die zeitlichen Vorteile durch die Schnellentlüftung nicht so zu erreichen wie beim erfindungsgemäßen System mit reibungsverlustarmen, großen Leitungs- und Schlauchdurchmessem.

Schließlich braucht die Druckblase/-Manschette nicht aus einer dehnfähigen Folie gefertigt zu werden, wie das bei den bisherigen marktüblichen Gummidruckblasen der Fall ist. Diese verlangen ein Dehnen, das für den Anwender beim manuellen Aufpumpen sehr anstrengend ist. Der Wirkungsgrad einer solchen Druckblase wird durch die teilweise Zweckentfremdung der Druckluftenergie für das Gummiblasendehnen deutlich verkleinert, weil diese Energie für den Prozess verloren geht. Hingegen kann für die zusammen mit der Ventile-Manometer-Einheit eingesetzte Druckblase/-manschette ein latexfreies Material zum Einsatz kommen, das leicht, kostengünstig und einfach verarbeitbar ist sowie in der täglichen Anwendung (Reinigung, Desinfektion ......) ein gutes Handling bietet. Dieses gute Handling gilt gleichermaßen für die Ventile-Manometer-Einheit, dessen aus Kunststoff gefertigter Korpus ebenfalls latexfrei ist, so daß keine toxischen Stoffe austreten können. Dabei ist er schlag- und kratzunempfindlich, spritzwasserfest, mittels Wischreinigung leicht zu reinigen und chemisch beständig, d.h. für die Reinigung mit krankenhausüblichen Reinigungs- und Desinfektionsmitteln - meist auf Formaldehyd- und Alkoholbasis - ausgelegt.

Weitere Merkmale und Einzelheiten der Erfindung ergeben sich aus dem Anspruch und der nachfolgenden Beschreibung eines in den nicht maßstäblichen Zeichnungen dargestellten Ausführungsbeispiels der Erfindung. Es zeigen:
Fig. 1 als Einzelheit in der Vorderansicht eines Ventile-Manometer-Einheit;
Fig. 2 die Ventile-Manometer-Einheit in einer Seitenansicht;
Fig. 3 einen Schnitt entlang der Linie III-III von Fig. 1;
Fig. 4 die Ventile-Manometer-Einheit mit daran angeschlossenem Pumpball und - explosiv dargestellt - Druckblase/-manschette, vor deren Aufstecken auf die Ventile-Manometer-Einheit;
Fig. 5 eine Schemazeichnung der Ventile-Manometer-Einheit mit Schaltbildern eines in ihr integrierten Schnellentlüftungsventils; und
Fig. 6 eine Schemazeichnung der Ventile-Manometer-Einheit wie zuvor, mit angedeutetem Rückschlagventil.

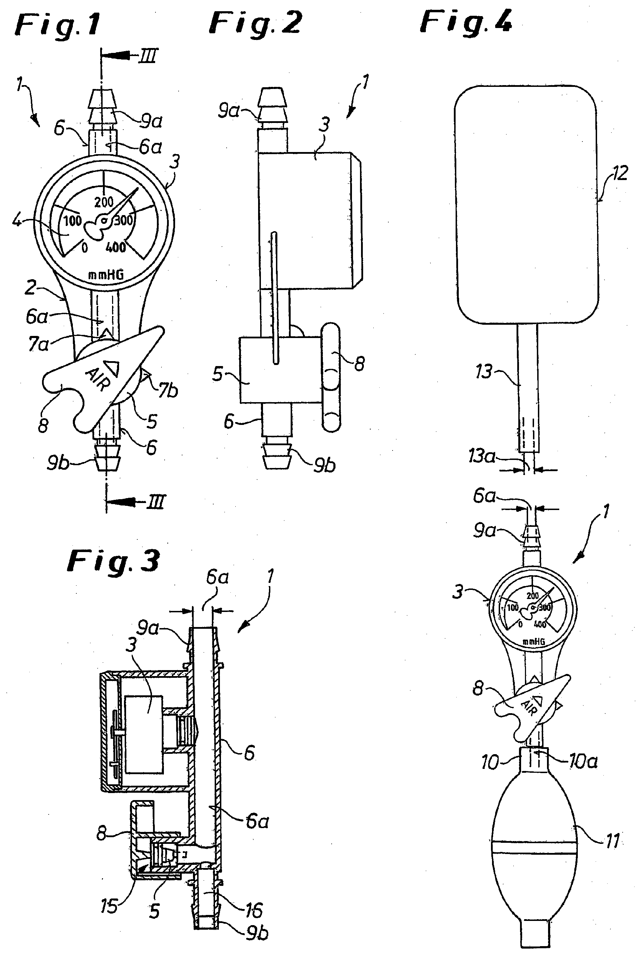

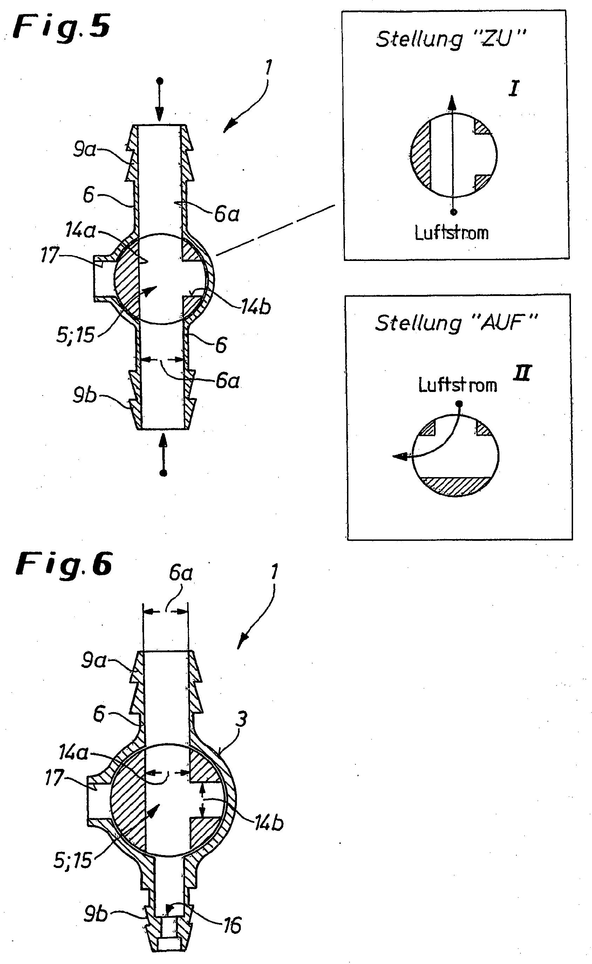

Eine in den Fig. 1 bis 3 dargestellte Ventile-Manometer-Einheit 1 besteht aus einem als kompaktes Kunststoff-Spritzgußteil hergestellten Gehäuse 2, das integriert ein auf Überdruckfestigkeit ausgelegtes Manometer (Rohrfedermanometer) 3 mit kratzunempfindlicher, schlagfester, glasklarer Sichtscheibe und eine in 100 mmHg-Schritten aufgedruckte Druckanzeige 4 sowie einen Drehzylinder 5 (vgl. auch die Fig. 5 und 6) aufnimmt, und ist mit einer diese Bauteile verbindenden Rohrleitung 6 ausgebildet.

Der in der Rohrleitung 6 angeordnete Drehzylinder 5 ist mit von außen ersichtlichen Pfeilen 7a bzw. 7b versehen, deren Pfeilspitze die Schaltstellungen "ZU" bzw. Stellung Aufpumpen (7a, I) und "AUF" bzw. Entlüften (7b, II) (vgl. auch Fig. 5) anzeigt, und über einen Drehhebel 8 von einem Bediener in die gewünschte Position zu verstellen ist. Die Rohrleitung 6 ragt beidendig, d.h. einander diametral gegenüberliegend mit Anschlussabschnitten 9a b zw. 9b aus dem Gehäuse 2 vor. Auf den Anschlussabschnitt 9b unterhalb des Drehzylinders 5 wird ein Schlauchende 10 eines Pumpballs 11 und auf den Anschlussabschnitt 9b oberhalb des Manometers 3 eine Druckblase bzw. -manschette 12 mittels eines Schlauchendes 13 aufgesteckt (vgl. Fig. 4).

Die Rohrleitung 6 der Ventile-Manometer-Einheit 1 einschließlich der Anschlussabschnitte 9a bzw. 9b besitzt einen durchgängig gleichen Innendurchmesser 6a, den somit auch die Öffnungsquerschnitte 14a bzw. 14b (vgl. die Fig. 5 und 6) des als Mehrwege-Schnellentlüftungsventil 15 ausgebildeten Drehzylinders 5 aufweien. Denselben großen, gleichen Innendurchmesser 13a bzw. 10a besitzen die Anschlussschlauchenden 13 der Druckblase 12 bzw. 10 des Pumpballs 11. Unterhalb des Mehrwege-Schnellentlüftungsventils 15 ist im Ausführungsbeispiel in dem Anschlussabschnitt 9b der Rohrleitung 6 ein Rückschlagventil 16 angeordnet (vgl. die Fig. 3 und 6); alternativ könnte dieses beispielsweise auch im Anschluß-Schlauchende 10 des Pumpballs 11 vorgesehen werden.

Zur Einsatzvorbereitung wird die Druckblase 12 mit einem darin zuvor lagesicher eingelegten Transfusions-/Infusionsbeutel (nicht dargestellt) aufgewickelt und mittels des Pumpballs 11 von Hand auf den notwendigen Betriebsdruck (300 mmHg) aufgepumpt. Der Drehhebel 8 des Drehzylinders 5 bzw. Schnellentlüftungsventils 15 wird hierzu in die Stellung I "ZU" bzw. Aufpumpen mit der in Fig. 1 dargestellten Richtungsanzeige des Pfeils 7a (vgl. auch Fig. 5 mit der oberen Einrahmung) gedreht. Der nach oben zeigende Pfeil 7a gibt diese Schaltposition an, in der die Luft aus dem Pumpball 11 durch das Rückschlagventil 16 und das Schnellentlüftungsventil 15 vorbei am Manometer 3 in das Schlauchende 13 der Druckblase 12 strömt und diese füllt. In dieser Schaltposition wird ein Druck von 300 mmHg weitgehend verlustfrei gespeichert. Die mit dem Schnellentlüftungsventil 15 zusammenwirkende Entlüftungsöffnung 17 der Ventile-Manometer-Einheit 1 ist druckdicht verschlossen (vgl. die Fig. 5 und 6). Das Rückschlagventil 16 ist so konstruiert, daß es von dem Luftstrom in Durchlassrichtung aufgrund einer großen effektiven Querschnittsfläche mit minimalem Strömungswiderstand passiert werden kann.

Wenn die Druckblase 12 ihren Einsatzzweck erfüllt hat und für einen weiteren Transfusions-/Infusionsbeutel vorbereitet werden soll, geschieht das schnellstmöglich dadurch, daß der Drehhebel 8 des Schnellentlüftungsventils 17 bzw. Drehzylinders 5 mit Richtung seines Pfeils 7b nach oben verdreht wird, so daß die restliche Druckluft aus der Druckblase 12 vorbei am Manometer 3 durch die seitliche Entlüftungsöffnung 17 nach außen entweichen kann (vgl. in Fig. 5 die untere Einrahmung Stellung "AUF" bzw. II). Auch hier ermöglichen die gleich großen Strömungsquerschnitte einen schnellen, ungehinderten Entlüftungsvorgang. Nach dem Einwickeln des weiteren Infusions-/Transfusionsbeutels kann die Druckblase bzw. -manschette 12 dann wie zuvor beschrieben sehr schnell wieder aufgepumpt werden.

## Patentansprüche

1. Vorrichtung zum Entleeren des Inhalts eines Transfusions-/Infusionsbeutels, umfassend einen Pumpball (11), ein Manometer (3), ein Schnellentlüftungsventil (5; 15), ein Rückschlagventil (16) und eine Druckblase (12), in die der Transfusions-Infusionsbeutel eingewickelt wird, wobei diese Bauteile über Schläuche miteinander verbindbar sind,
**gekennzeichnet durch**
eine Ventile-Manometer-Einheit (1), die in einem gemeinsamen Gehäuse (2) integriert das Manometer (3) und das Schnellentlüftungsventil (5; 15) aufweist, die über eine Rohrleitung (6) miteinander verbunden sind, deren Innendurchmesser (6a) einen durchgängig einheitlich großen Strömungsquerschnitt wie auf beldendige Anschlussabschnitte (9a bzw, 9b) der Rohrleitung (6) aufzusteckende Schlauchenden (10 bzw. 13) einerseits des Pumpballs (11) bzw. andererseits der Druckblase (12) besitzt, wobei in der Rohrleitung (6) zwischen dem Pumpball-Anschlussabschnitt (9b) und dem Manometer (3) ein Drehzylinder (5) als Mehrwege-Schnellentlüftungsventil (15) angeordnet Ist, das in den Ventilstellungen (I bzw. II) zum Aufpumpen (I) oder zum Entlüften (II) mit dem durchgängig einheitlich großen Strömungsquerschnitt oder Innendurchmesser (14a bzw, 14b) wie die Rohrleitung (6), die das Manometer und das Schnellentlüftungsventil (5; 15) miteinander verbindet, ausgebildet ist, und in dem Pumpball-Anschlussabschnitt (9b; 10) das Rückschlagventil (16) vorgesehen ist.

## Claims

1. A device for emptying the contents of a transfusion/infusion sac, comprising a pumpball (11), a manometer (3), a rapid action ventilation valve (5; 15), a non-return valve (16) and a pressure bladder (12), in which the transfusion/infusion sac is enveloped, wherein said components may be connected to each other by means of tubes,
**characterised by**
a valve-manometer unit (1), comprising the manometer (3) and the quick action ventilation valve (5; 15) integrated in a common housing (2) and connected to each other by means of a tube line (6), of which the inner diameter (6a) has the same continuously uniformly sized flow cross section as tube ends (10 and 13) on the one hand of the pumpball (11) and on the other hand of the pressure bladder (12), said tube ends being intended for respective connection to connector sections (9a and 9b) at both ends of the tube line (6), wherein a rotating cylinder (5) is arranged as a multiple path rapid-action ventilation valve (15) in the tube line (6) between the pumpball connector section (9b) and the manometer (3), said ventilation valve, in the valve positions (I and II) for pumping (I) or for ventilation (II), being embodied with the same continuously uniformly sized flow cross section or inner diameter (14 and 14b) as the tube line (6) interconnecting the manometer and the quick-action ventilation valve (5; 15), and the non-return valve (16) is provided in the pumpball connector section (9b; 10).

## Revendications

1. Procédé pour vider le contenu d'une poche de transfusion/perfusion, comprenant une pompe à ballonnet (11), un manomètre (3), une soupape d'évacuation rapide (5 ; 15), un clapet anti-retour (16) et une vessie (12) dans laquelle la poche de transfusion/perfusion est enroulée, sachant que ces pièces sont reliées entre elles par des tuyaux, **caractérisé par** une unité de soupape-manomètre (1) qui présente le manomètre (3) et la soupape d'évacuation rapide (5 ; 15) intégrés dans un boîtier commun (2), qui sont reliés entre eux par un tube (6) dont le diamètre intérieur (6a) possède une section d'écoulement toujours uniformément égale à des extrémités de tuyau (10, respectivement 13) à placer sur des tronçons d'extrémité (9a, respectivement 9b) à deux extrémités du tube (6), d'un côté de la pompe à ballonnet (11), respectivement de l'autre côté de la vessie (12), sachant que dans le tube (6) est disposé un cylindre rotatif (5) en tant que soupape d'évacuation rapide-multivoies (15) entre le tronçon d'extrémité (9b) de la pompe à ballonnet et le manomètre (3), lequel est prévu pour pomper (I) dans les positions de soupape (I, respectivement II) ou pour évacuer (II) avec la section d'écoulement toujours uniformément égale ou le diamètre intérieur (14a, respectivement 14b) au tube (6) qui relie entre eux le manomètre et la soupape d'évacuation rapide (5 ; 15), et le clapet anti-retour (16) est prévu dans le tronçon d'extrémité (9b ; 10) de la pompe à ballonnet.
